(19) Europäisches Patentamt
European Patent Office
Office européen des brevets

(11) EP 0 971 692 B1

(12) **EUROPÄISCHE PATENTSCHRIFT**

(45) Veröffentlichungstag und Bekanntmachung des Hinweises auf die Patenterteilung:
**05.03.2003 Patentblatt 2003/10**

(21) Anmeldenummer: **98919046.7**

(22) Anmeldetag: **06.03.1998**

(51) Int Cl.[7]: **A61K 9/00**

(86) Internationale Anmeldenummer:
**PCT/DE98/00668**

(87) Internationale Veröffentlichungsnummer:
**WO 98/040049 (17.09.1998 Gazette 1998/37)**

(54) **SPEZIFISCHE MAGNETOSOMEN, VERFAHREN ZU IHRER HERSTELLUNG UND IHRE VERWENDUNG**

SPECIFIC MAGNETOSOME, METHOD FOR THE PRODUCTION AND USE THEREOF

MAGNETOSOMES SPECIFIQUES, LEUR PROCEDE DE PRODUCTION ET LEUR UTILISATION

(84) Benannte Vertragsstaaten:
**AT BE CH DK ES FI FR GB IT LI NL SE**

(30) Priorität: **07.03.1997 DE 19709322**
**14.04.1997 DE 19716732**

(43) Veröffentlichungstag der Anmeldung:
**19.01.2000 Patentblatt 2000/03**

(73) Patentinhaber:
• **MAX-DELBRÜCK-CENTRUM FÜR MOLEKULARE MEDIZIN**
**13125 Berlin (DE)**
• **Max-Planck-Gesellschaft zur Förderung der Wissenschaften e.V. Berlin**
**80539 München (DE)**

(72) Erfinder:
• **BÄUERLEIN, Edmund**
**D-81375 München (DE)**
• **SCHÜLER, Dirk**
**D-39418 Sta furt (DE)**
• **RESZKA, Regina**
**D-16341 Schwanebeck (DE)**

• **PÄUSER, Sabine**
**D-12435 Berlin (DE)**

(74) Vertreter: **Baumbach, Friedrich, Dr.**
**Patentanwalt**
**Robert-Rössle-Strasse 10**
**13125 Berlin (DE)**

(56) Entgegenhaltungen:
**US-A- 4 677 067**

• **Chem. abstr., Band 127, Nr. 11, 15. September 1997 (Columbus, Ohio, USA), Seiten 279-280, Zusammen- fassungs-Nr. 146865y, SCHUELER, D. et al. "Iron transport and magnetite crystal formation of the magnetic bacterium Magneto-spirillum gryphiswaldense", XP 002900173; J. Phys. IV 1997, 7(C1, 7th International Conference on Ferrites, 1996), C1/647- -C1/650 (Eng).**
• **Chem. abstr., Band 109, Nr. 20, 14. November 1988 (Columbus, Ohio, USA), Seite 383, linke Spalte, Zusammenfassungs-Nr. 176329x, GOMIYO, H. et al. "Liposomes containing magnetite", XP002900174 & JP 63 014717 A (88 14,717).**

**Beschreibung**

Die Erfindung betrifft spezifische Magnetosomen mit Magnetpartikeln von maximal 43-45 nm, Verfahren zu ihrer Herstellung und ihre Verwendung. Sie betrifft ferner Magneto-Liposomen, die aus diesen Magnetosomen durch liposomale Verkapselung erhalten werden.

Anwendungsgebiete der Erfindung sind die Medizin und die pharmazeutische Industrie.

Es ist bekannt, daß superparamagnetische Eisenpartikel in der medizinischen Diagnostik als NMR-Kontrastmittel oder in Form von Immuno-Konjugaten oder als synthetische Drug-Carrier angewendet werden. Matsunaga et al. beschrieben 1989 Magnetosomen, gewonnen aus dem magnetischen Bakterium *Magnetospirillum* spec. ABM1 (JP7-241192-A), sowie ihre Anwendung. Diese Magnetosomen haben jedoch den Nachteil, daß sie relativ groß sind und somit die Gefahr besteht, daß sie bei medizinischer Anwendung Embolien auslösen können.

Es war deshalb Aufgabe der Erfindung, spezifische Magnetosomen bereitzustellen, die kleiner als die bekannten sind, wodurch die

- medizinische Anwendbarkeit hinsichtlich der Erreichbarkeit von vorgesehenen Zielen im Körper des Patienten verbessert und
- gleichzeitig die Gefahr von Embolien verringert wird.

Es wurde gefunden, daß Magnetosomen mit Magnetpartikeln < 50 nm im Bakterium *Magnetospirillum gryphiswaldense* enthalten sind.

Überraschend konnten diese spezifischen Magnetosomen aus dem magnetischen Bakterium *Magnetospirillum gryphiswaldense* in halbtechnischem Maßstab hergestellt werden.

Gegenstand der Erfindung sind danach die Magnetosomen selbst, ihr Herstellungs-/Gewinnungsverfahren und ihre Verwendung, bevorzugt in der Medizin und in der Pharmazie.

Die Erfindung wird gemäß den Ansprüchen realisiert.

Die erfindungsgemäßen Magnetosomen sind durch einen Einzelkristall des magnetischen Eisenoxids Magnetit $Fe_3O_4$ mit einem maximalen Durchmesser von 43-45 nm, der von einer Phospholipidmembran umgeben ist, gekennzeichnet. Sie haben in der Regel eine kubooktaedrische Form.

Vorzugsweise besteht die Membran aus Phosphatidylethanolamin, Phosphatidylglycerol und Phosphatidylcholinen, in denen hauptsächlich die Fettsäuren Palmitinsäure, Palmitoleinsäure und Ölsäure vorhanden sind. Besonders bevorzugt setzt sich die Membran aus $53 \pm 6\%$ Phosphatidylethanolamin, $38 \pm 6\%$ Phosphatidylglycerol und $8,9 \pm 0,5\%$ Phosphatidylcholine zusammen, in denen sich hauptsächlich die Fettsäuren Palmitinsäure (ca. 18,4%), Palmitoleinsäure (ca. 25,6%) und Ölsäure (ca. 45,9%) befinden.

In einer bevorzugten Ausführungsvariante liegen die Magnetosomen als Ketten von bis zu 100, bevorzugt 10-60, Magnetosomen und mit kationischer Oberflächenladung vor. Durch diese Kettenform der Magnetosomen wird die Wahrscheinlichkeit erhöht, daß Antikörper und Therapeutika korrekt an sie binden und wirken können.

Erfindungsgemäße Magnetosomen sind außerdem auch Magnetosomen, die zusätzlich kovalent gebundene Antikörper oder Therapeutika aufweisen, die über entsprechende reaktive Gruppen an die Magnetosomenmembran gebunden sind.

Die Erfindung betrifft darüber hinaus ein Verfahren zur Herstellung dieser neuen Magnetosomen. Sie werden aus dem magnetischen Bakterium *Magnetospirillum gryphiswaldense* nach einem neuen Fermentationsverfahren isoliert. Dazu wird ein neues, einfaches Kulturmedium, bestehend aus 0,3 g $KH_2PO_4$, 1 g Na-Acetat, 1 g Soja-Pepton (Merck), 0,1 g 0.1 $NH_4Cl$, 0,1 g Hefeextrakt, pH 6.9 bevorzugt verwendet, das keinen Komplexbildner für Eisen enthält. Die Sauerstoffkonzentration wird im Medium unterhalb von 2 % gehalten, später werden Na-Acetat sowie $FeSO_4$ zugesetzt. Nach ca. 30 Stunden können die magnetischen Zellen geerntet werden. Nach Zellyse werden die Magnetosomen durch ein neues Verfahren in hoher Ausbeute gewonnen, in dem sie in einer magnetischen Separationssäule mit einem starken kräftigen Permanentmagneten (Sm-Neodyn) von Zelltrümmern und Zellsaft abgetrennt und durch Waschen gereinigt werden.

Gegenstand der Erfindung sind weiterhin erfindungsgemäße Magnetosomen, die in Liposomen verpackt vorliegen, mit anderen Lipiden selbst Liposomen bilden oder an die Oberfläche von Liposomen gebunden sind. Solche Liposomen sind

- sogenannte klassische Liposomen (MLV, SUV,LUV)
- sogenannte "Stealth"-Liposomen (PEG)
- Micellare Systeme (z.B. SDS, Triton, Natriumcholat)
- Immunoliposomen, die z. B. Antikörper oder Fab-Fragmente gegen krankheitsassoziierte Antigene bzw. Adhäsionsmoleküle, an die Oberfläche der Liposomen gebunden, enthalten
- sogenannte kationische Liposomen (DAC-Chol, DOCSPER)

- sogenannte fusogene Liposomen (rekonstituierte Fusionsproteine in Liposomen).

Zur Herstellung dieser Magneto-Liposomen werden die an sich bekannten Liposomen-Herstellungsmethoden verwendet, z. B. beschrieben in DE 41 34 158, DE 44 30 593, DE 44 46 937 und DE 196 31 189, wobei die Magnetosomen bevorzugt zu den Ausgangslipiden zugesetzt werden.

Die zur Erfindung gehörenden bevorzugten Modifikationen von Magneto-Liposomen und Magnetosomen sind in nachfolgender Tabelle 1 dargestellt.

```
Tabelle 1
```

```
                          Magneto-Liposomen
                                  ↓
    Klassische "Stealth"    Immuno    Kationische    Fusogene
                                                z.B.      z.B.
    MLV            PEG      anti CEA    DAC-Chol/DOPE  HN-,F-Protein
    SUV                     anti Thy1.1 SP-Chol/DOPE  (SendaiVirus)
    LUV                     anti CD44   DAC-Quat.-      → [pH 7]
    (REV)                   anti CD54   Chol/DOPE     Synthetische
                            anti CD56   DOCSPER       Fusions-
                            anti CD30                 proteine
                            anti CD31                 HA(Influenza-
                                                      virus)[pH5,2]
                                                      "Cochleates"
```

```
                          Magnetosomen
                                  ↓
        Immuno               Gen-bzw. Antisense Oligonukleotid,
        anti CEA             oder Ribozym modifizierte
        anti CD44
        anti CD54, CD56
        anti CD30
```

Die erfindungsgemäßen Magnetosomen und Magneto-Liposome können zusätzlich an ihren Oberflächen chemisch gekoppelte spezifische Antikörper, ein oder mehrere Therapeutika und Radionuklide eingeschlossen, das heißt verkapselt, enthalten.

Außerdem können sie zusammen mit genetischem Material, wie z.B. Plasmiden, Therapiegenen, Antisense-Oligonukleotiden, Ribozymen oder Gendiagnostika kationische Komplexe bilden, die zum Gentransfer geeignet sind.

Diese erfindungsgemäßen Magnetosomen und Magneto-Liposomen haben ein umfangreiches Anwendungsspektrum. Aufgrund ihrer magnetischen Eigenschaften werden sie als solche (also unmodifiziert) als Kontrastmittel für NMR-Untersuchungen und als Marker zum Mapping der magnetischen Suszeptibilitäten durch ein SQUID Biomagnetometer und auch als Diagnostika zur Detektion verschiedener Krankheiten und Entzündungsherde oder als Therapeutika angewendet wie z. B. zum Purging ("Herausfischen von Krankheitszellen", als Diagnostika für Tumorerkrankungen bzw. Lymphographie, für entzündliche Prozesse, für multiple Sklerose, Alzheimersche Krankheit und für die Parkinson-Erkrankung oder als Therapeutikum gegen Tumorerkrankungen, endzündliche Prozesse sowie Stoffwechselerkrankungen.

Der Einsatz als Diagnostika erfolgt vorzugsweise in Form von Immuno-Magnetosomen oder Immuno-Magneto-Liposomen. Dazu sind Antikörper bzw. Fab-Fragmente gegen krankheitsassoziierte Antigene bzw. Adhäsionsmoleküle oder Liganden über entsprechende Gruppen kovalent an die Magnetosomen- oder Magneto-Liposomen-Membran gekoppelt, bevorzugt über Spacer verschiedener Länge an das in der Membran enthaltene Phosphatidylethanolamin.

Sie werden insbesondere als Diagnostika zur Detektion von Tumorerkrankungen bzw. zur Lymphographie eingesetzt, wobei u. a. anti-CEA, anti-CD44, als Reagenz an die Magnetosomen-Membran bzw. Magneto-Liposomen-Membran gekoppelt sind.

Auch zur Detektion entzündlicher Prozesse wie Arthrosen (bevorzugt mit Anti CD54, Anti CD 56) oder zur Erkennung von multipler Sklerose bzw. der Alzheimerschen Krankheit (bevorzugt anti-β-Amyloid, anti-APOE4), von Hogkin-Lymphomzellen (bevorzugt mit Anti-CD 30) und der Parkinson-Erkrankung sind diese Antikörper-Koppelungsprodukte geeignet.

Für die genannten diagnostischen Verwendungen sind die erfindungsgemäßen Magnetosomen hervorragend geeignet.

Um gleichzeitig eine therapeutische Substanz in relevanten Mengen an den Zielort zu bringen, ist es notwendig, Magneto-Liposomen einzusetzen. Sie sind nicht nur für die Ankoppelung, sondern auch für einen Einschluß von Therapeutika geeignet. Im Falle von Magnetosomen können Therapeutika nur unter Zwischenschaltung eines Spacers angekoppelt werden.

Eine wesentliche Verwendungsmöglichkeit gemäß der Erfindung besteht darin, daß Therapeutika angekoppelt (Magnetosomen) bzw. angekoppelt oder eingeschlossen (Magneto-Liposomen) werden. Diese Therapeutika können je nach Lipophilie oder Hydrophilie in der Membran bzw. im wäßrigen Innenraum der Liposomen eingeschlossen werden.

Damit ergeben sich erfindungsgemäß folgende bevorzugten Kopplungsvarianten:

- das oder die Therapeutika sind an das Magnetosom gekoppelt oder in der Membran eingeschlossen,
- das oder die Therapeutika sind an das Magnetosom gekoppelt oder in der Membran eingeschlossen und in Liposomen verpackt,
- das Magnetosom ist als Liposomen verpackt und das oder die Therapeutika sind im wäßrigen Innenraum der Liposomen eingeschlossen,
- Therapeutika sind an das Magnetosom gekoppelt oder in der Membran eingeschlossen, das Magnetosom ist in Liposomen verpackt und mindestens ein weiteres Therapeutikum ist im wäßrigen oder lipophilen Innenraum der Liposomen eingeschlossen.

Wichtige in Betracht kommende Therapeutika sind Chemotherapeutika wie Carboplatin oder Taxol und Radiotherapeutika wie Ytrium, Jod, Technetium oder Bor. Ebenso kann man Therapiegene wie Suizidgene, Antisense-Oligonukleotide, Ribozyme oder Cytokingene ankoppeln.

Mit der Erfindung wird eine breite medizinische Anwendung ermöglicht. Der wesentliche Vorteil der erfindungsgemäßen Magnetosomen und Magnetoliposomen besteht darin, daß

- Metastasen im Körper besser erreicht und frühzeitig entdeckt werden können,
- eine verbesserte Anreicherung in den Lymphgefäßen erfolgt
- mit den neuen Partikeln eine bessere Blut-Hirnschranken-Gängigkeit erreicht wird, was insbesondere zur Detektion von Alzheimer-Plaques und zur Diagnose von Hirntumoren von Bedeutung ist.

Anschließend wird die Erfindung an Ausführungsbeispielen näher erläutert.

1. Gewinnung der Magnetosomen

Zur massenhaften Gewinnung der Magnetosomen wurden die Zellen des magnetischen Bakteriums Magnetospirillum gryphiswaldense bei 30 °C in einem 100 l Fermenter (LP 352, Bioeng. AG) im Kulturmedium folgender Zusammensetzung angezüchtet (per 1000 ml): 0,3 g $KH_2PO_4$, 1 g Na-Acetat, 1 g Soja-Pepton (Merck), 0,1 g 0.1 $NH_4Cl$, 0,1 g Hefeextrakt, pH 6.9. Die Beimpfung erfolgte durch Zugabe einer 5 l Vorkultur zu 70 l Medium. Die Regulierung der Belüftung erfolgte über die Rührung und Druckluftzufuhr, so daß die Sauerstoffkonzentration im Medium 2 % Sättigung nicht überschritt. Bei einer $OD_{400}$=0,55 wurden 70 g Na-Acetat sowie $FeSO_4$ zu einer Konzentration von 100 µM zugesetzt. Nach ca. 30 Stunden konnten die magnetischen Zellen geerntet werden.

Die Zellen wurden herunterzentrifugiert und gewaschen. Nach dreimaliger Passage durch die French Press und nachfolgender niedertouriger Zentrifugation wurde der Zellextrakt in 20 mM HEPES/4 mM EDTA über eine magnetische Separationssäule (Miltenyi Biotec) gegeben. Zur Abtrennung der magnetischen Partikel wurde die Säule dem Magnetfeld eines kräftigen Permanentmagneten (Sm-Neodyn) ausgesetzt. Dies erzeugte ein starkes inhomogenes Magnetfeld im magnetisierbaren Säulenmaterial somit zur spezifischen Bindung der magnetischen Partikel. Die Magnetosomen wurden auf der Säule mit 20 mM HEPES/200 mM NaCl gewaschen, um spezifisch assoziierte Verunreinigungen zu entfernen. Nach Waschen mit 20 mM HEPES wurden die Magnetosomen nach Entfernen des Magnetfelds von der Säule gespült. Zur Abtrennung von möglicherweise vorhandenen Membranverunreinigungen wurde die Magnetosomensuspension auf einen zweistufigen (50/55 % Sacharose) Zuckergradienten aufgetragen und 25 h in der Ultrazentrifuge bei 25 T rpm zentrifugiert. Eventuell vorhandene Mermbranbestandteile sammelten sich an der Übergangsphase Puffer-Sacharose-Lsg, während die Magnetosomenpartikel als Pellet am Boden des Röhrchens erschienen. Die so gewonnenen Magnetosomen erschienen elektronenmikroskopisch rein und wiesen ein distinktes Lipid- und Proteinmuster auf.

2. Verwendung der Magnetosomen

Magnetosomen aus M. gryphiswaldense mit einem Eisengehalt (mit der Atomabsorptionsspektroskopie (AAS) ermittelt) von 1.35 g Fe/l wurden eingesetzt.

Die Relaxivitäten wurden an einem Bruker Minispec pc 120 bei 37°C und 0.47 T bestimmt zu:

$R_1$ = 25.503 $mM^{-1} * s^{-1}$
$R_2$ = 226.179 $mM^{-1} * s^{-1}$.

Diese Relaxivitäten besonders die $R_2$ sind im Vergleich zu verschiedenen SPIO'S (Superparamagnetic Ironoxid-Formulierungen) hoch. Nur bei den SPIO-SUV's (small unilamellar vesicles) wurden vergleichbare Werte erzielt. Folgendes in-vivo-Experiment wurde durchgeführt: Einer männlichen WAG/RIJ (270 g) mit einem in die Leber implantierten CC531 Adenokarzinom wurde i.v. in der Schwanzvene die restlich verbliebene Substanzmenge (0,4 ml) injiziert. Das Tier erhielt somit die Magnetosomen in einer Dosis von 35,81 µmol Fe/kg Rattengewicht. Die NMR-Untersuchung erfolgte an einem Bruker Biospec BMT 24/40. Dabei wurden vor, sofort nach Injektion und dann zu den in der Tabelle 2 angegebenen Zeitpunkten neun 3 mm dicke Schichten durch das Abdomen der Ratte und ein beigefügtes externes Standardröhrchen mit der RARE-Sequenz (TR=2500 ms, TE=20 ms, RF=8; NE=8) aufgenommen. Die Signalintensitäten in Leber und Tumor wurden in vier verschiedenen Schichten gemessen und ausgewertet. Die angegebene Schwächung der relativen Signalintensität $SI_{rel}$ berechnet sich folgendermaßen:

$$SI_{rel.} = (SI_{post\ Lip..}/SI_{Standard})/(SI_{pre\ LIP}/SI_{Standard})$$

$SI_{pre\ Lip}$ = Signalintensität vor Applikation der Liposomen
$SI_{post\ Lip.}$ = Signalintensität nach Applikation der Liposomen
$SI_{Standard}$ = Signalintensität des Standardes

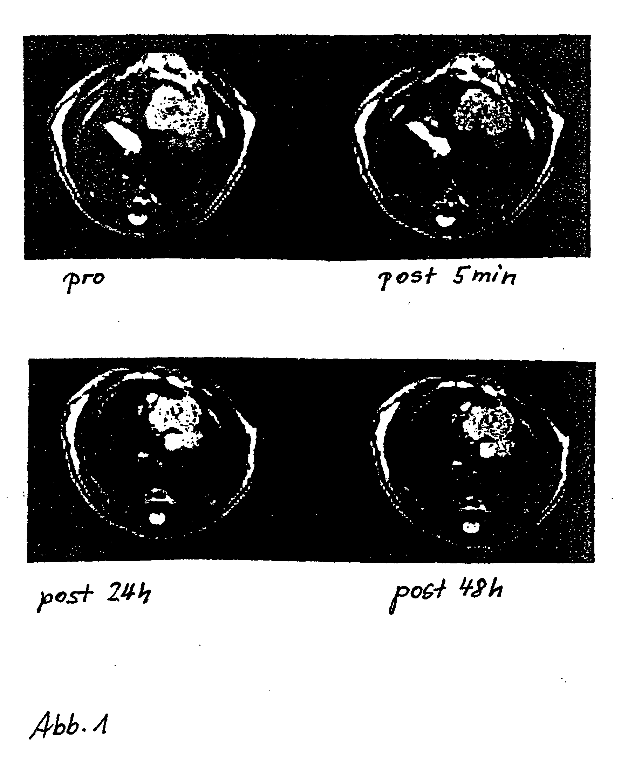

In der Leber wurde schon bei dieser verhältnismäßig geringen Dosis eine Signalreduktion von bis zu 90% erzielt, im Tumor wurden jedoch nur schwache SI-Reduktionen erzielt (Tabelle 2). Das bedeutet, daß sich der Tumor klar von gesundem Lebergewebe abhebt (Abb.1).

Tabelle 2

| | Tumor Mittelwert aus allen > Schichten | | | | Mittelwert | |
|---|---|---|---|---|---|---|
| | | | | | | |
| pre | 1,00 | 1,00 | 1,00 | 1,00 | 1,00 | 0,00 |
| 5 min | 0,93 | 0,89 | 0,91 | 0,98 | 0,93 | 0,04 |
| 15 min | 1,00 | 0,96 | 0,98 | 1,02 | 0,99 | 0,03 |
| 31 min | 1,04 | 0,99 | 0,99 | 1,06 | 1,02 | 0,04 |

Tabelle 2 (fortgesetzt)

| | | Tumor Mittelwert aus allen > Schichten | | | Mittelwert | |
|---|---|---|---|---|---|---|
| 48 min | 1,01 | 0,98 | 0,98 | 1,06 | 1,00 | 0,04 |
| 65 min | 1,00 | 0,98 | 0,98 | 1,13 | 1,02 | 0,07 |
| 82 min | 0,95 | 0,94 | 0,93 | 1,05 | 0,97 | 0,06 |
| 113 min | 0,93 | 0,86 | 0,91 | 1,10 | 0,95 | 0,10 |
| 24 h | 0,93 | 0,86 | 0,91 | 1,10 | 0,95 | 0,10 |
| 48 h | 1,05 | 1,00 | 1,02 | 1,14 | 1,05 | 0,10 |
| 110 h | 0,81 | 0,73 | 0,85 | 0,72 | 0,78 | 0,06 |
| | Leber | | | | Mittelwert | Standardabweichung |
| pre | 1,00 | 1,00 | 1,00 | 1,00 | 1,00 | 0,00 |
| 5 min | 0,18 | 0,29 | 0,13 | 0,12 | 0,18 | 0,08 |
| 15 min | 0,19 | 0,35 | 0,10 | 0,13 | 0,19 | 0,11 |
| 31 min | 0,13 | 0,19 | 0,09 | 0,15 | 0,14 | 0,04 |
| 48 min | 0,18 | 0,14 | 0,14 | 0,23 | 0,17 | 0,04 |
| 65 min | 0,24 | 0,18 | 0,14 | 0,13 | 0,17 | 0,05 |
| 82 min | 0,23 | 0,13 | 0,11 | 0,17 | 0,16 | 0,05 |
| 113 min | 0,13 | 0,13 | 0,11 | 0,17 | 0,13 | 0,03 |
| 24 h | 0,11 | 0,13 | 0,11 | 0,17 | 0,13 | 0,03 |
| 48 h | 0,11 | 0,12 | 0,12 | 0,11 | 0,11 | 0,01 |
| 110 h | 0,13 | 0,11 | 0,12 | 0,13 | 0,12 | 0,01 |

## Patentansprüche

1. Spezifische Magnetosomen bestehend aus einem Einzelkristall des magnetischen Eisenoxids Magnetit $Fe_3O_4$ mit einem Durchmesser ≤ 45 nm und einer diesen Kristall umgebenden Phospholipidmembran.

2. Magnetosomen nach Anspruch 1, **dadurch gekennzeichnet, daß** die Membran aus Phosphatidylethanolamin, Phosphatidylglycerol und Phosphatidylcholin besteht, in denen hauptsächlich die Fettsäuren Palmitinsäure, Palmitoleinsäure und Ölsäure vorhanden sind.

3. Magnetosomen nach Anspruch 1 und 2, **dadurch gekennzeichnet, daß** die Membran sich aus 53 ± 6% Phosphatidylethanolamin, 38 ± 6% Phosphatidylglycerol und 8,9 ± 0,5% Phosphatidylcholinen zusammensetzt.

4. Magnetosomen nach Anspruch 1 bis 3, **dadurch gekennzeichnet, daß** sie als Ketten von bis zu 100, vorzugsweise 10-60, Magnetosomen und mit kationischer Oberflächenladung vorliegen.

5. Magnetosomen nach Anspruch 1 bis 4, **dadurch gekennzeichnet, daß** zusätzlich Antikörper oder Therapeutika kovalent, ggf. über entsprechende reaktive Gruppen, an die Magnetosomen-Membran gebunden sind.

6. Magnetosomen nach Anspruch 1 bis 5, **dadurch gekennzeichnet, daß** sie in Liposomen verpackt vorliegen.

7. Magnetosomen nach Anspruch 1 bis 6, **dadurch gekennzeichnet, daß** sie in klassischen Liposomen, Stealth-Liposomen, micellären Systemen, Immunoliposomen, kationischen Liposomen oder fusogenen Liposomen verpackt vorliegen.

8. Magnetosomen nach Anspruch 1 - 4 und 6 - 7, **dadurch gekennzeichnet, daß** sie zusätzlich an ihrer Oberfläche chemisch gekoppelte spezifische Antikörper aufweisen.

9. Magnetosomen nach Anspruch 1 - 4 und 6 - 7, **dadurch gekennzeichnet, daß** sie zusätzlich ein oder mehrere Therapeutika eingeschlossen enthalten (Verkapselung).

10. Magnetosomen nach Anspruch 1 - 4 und 6 - 7, **dadurch gekennzeichnet, daß** sie zusätzlich Radionuklide eingeschlossen enthalten (Verkapselung).

11. Magnetosomen nach einem der Ansprüche 1 - 4 und 6 - 7, **dadurch gekennzeichnet, daß** sie zusammen mit genetischem Material (z. B. Plasmiden) Therapiegenen, Antisense-Oligonukleotiden, Ribozymen oder Gendiagnostika zum Gentransfer geeignete kationische Komplexe bilden.

12. Verfahren zur Herstellung von spezifischen Magnetosomen gemäß Anspruch 1 bis 4, **dadurch gekennzeichnet, daß** man sie aus dem magnetischen Bakterium *Magnetospirillum gryphiswaldense,* unter Verwendung eines einfachen Kulturmediums, das keine Komplexbildner für Eisen enthält, isoliert, wobei man die Sauerstoffkonzentration im Medium unterhalb von 2 % hält, später Na-Acetat sowie $FeSO_4$ zusetzt, die magnetischen Zellen durch Zentrifugation erntet und anschließend nach Zellyse die Magnetosome durch Abtrennen von Zelltrümmern und Zellsaft mit einem Permanentmagneten in einer magnetischen Separationssäule gewinnt.

13. Verwendung von spezifischen Magnetosomen gemäß Anspruch 1 - 4 und 6-7 als NMR-Kontrastmittel.

14. Verwendung von spezifischen Magnetosomen gemäß Anspruch 1 bis 5 zu Herstellung von Arzneimitteln zum Purging ("Herausfischen von Krankheitszellen").

15. Verwendung von spezifischen Magnetosomen gemäß Anspruch 1 - 4 und 6 - 7 zu Herstellung von Diagnostika für Tumorerkrankungen bzw. Lymphographie, für entzündliche Prozesse, für multiple Sklerose, Alzheimersche Krankheit und für die Parkinson-Erkrankung.

16. Verwendung von spezifischen Magnetosomen gemäß Anspruch 1 bis 10 zu Herstellung von Therapeutika gegen Tumorerkrankungen, entzündliche Prozesse sowie Stoffwechselerkrankungen.

**Claims**

1. Specific magnetosomes comprising a single crystal of the magnetic iron oxide magnetite $Fe_3O_4$ with a diameter $\leq 45$ nm and a phospholipid membrane surrounding said crystal.

2. Magnetosomes according to Claim 1, wherein the membrane comprises phosphatidylethanolamine, phosphatidylglycerol and phosphatidylcholine, in which predominantly the fatty acids palmitine acid, palmitoleic acid and oleic acid are present.

3. Magnetosomes according to Claims 1 and 2, wherein the membrane is composed of $53 \pm 6$ % phosphatidylethanolamine, $38 \pm 6$ % phosphatidylglycerol and $8.9 \pm 0.5$ % phosphatidylcholines.

4. Magnetosomes according to Claims 1 to 3, wherein they are available as chains of up to 100, preferably 10-60 magnetosomes and with a cationic surface charging.

5. Magnetosomes according to Claims 1 to 4, wherein antibodies or therapeutic agents are additionally bound to the magnetosome membrane covalently, if need be via corresponding reactive groups.

6. Magnetosomes according to Claims 1 to 5, wherein they are available packed in liposomes.

7. Magnetosomes according to Claims 1 to 6, wherein they are available packed in classical liposomes, stealth liposomes, micellar systems, immunoliposomes, cationic liposomes or fusogenic liposomes.

8. Magnetosomes according to Claims 1 to 4 and 6 to 7, wherein they additionally manifest specific antibodies chemically coupled to their surface.

9. Magnetosomes according to Claims 1 to 4 and 6 to 7, wherein they additionally contain one or a plurality of enclosed therapeutica (encapsulation).

10. Magnetosomes according to Claims 1 to 4 and 6 to 7, wherein they additionally contain radio nuclides enclosed (encapsulation).

11. Magnetosomes according to Claims 1 to 4 and 6 to 7, wherein they form cationic complexes suited to gene transfer together with genetic material (e.g. plasmides), therapy genes, anti-sense oligonucleotides, ribozymes or gene diagnostica.

12. Method for the production of specific magnetosomes according to Claims 1 to 4, wherein they are isolated from the magnetic bacterium *Magnetospirillum gryphiswaldense* making use of a simple culture medium not containing any complex formers for iron, with the oxygen concentration in the medium being kept under 2 %, Na acetate and FeSO4 later being added, the magnetic cells being harvested by centrifuging and subsequently the magnetosomes being obtained following cell lysis by separation from cell debris and cell juice with a permanent magnet in a magnetic separation column.

13. Use of specific magnetosomes according to Claims 1 to 4 and 6 to 7 as NMR contrast agents.

14. Use of specific magnetosomes according to Claims 1 to 5 for the production of medications for purging ("fishing out disease cells").

15. Use of specific magnetosomes according to Claims 1 to 4 and 6 to 7 for the production of diagnostica for tumour diseases or lymphography, for inflammatory processes, for multiple sclerosis, Morbus Alzheimer and for Morbus Parkinson.

16. Use of specific magnetosomes according to Claims 1 to 10 for the production of therapeutica against tumour diseases, inflammatory processes as well as diseases of the metabolism.


**Revendications**

1. Magnétosomes spécifiques composés d'un seul cristal d'oxyde de fer magnétique, de magnétite $Fe_3O_4$, d'un diamètre $\leq$ 45 nm et d'une membrane phospholipide enveloppant ce cristal.

2. Magnétosomes selon la revendication 1, se caractérisant par le fait que la membrane se compose de phosphatidyléthanolamine, de phosphatidylglycérol et de phosphatidylcholine qui contiennent pour l'essentiel les acides gras suivants: acide palmitique, acide palmito-oléique et acide oléique.

3. Magnétosomes selon la revendication 1 et 2, se caractérisant par le fait que la membrane se compose de $53 \pm 6$ % de phosphatidyléthanolamines, de $38 \pm 6$ % de phosphatidylglycérols et de $8,9 \pm 0,5$ % de phosphatidylcholines.

4. Magnétosomes selon la revendication 1 à 3, se caractérisant par le fait qu'ils se présentent sous forme de chaînes comportant jusqu'à 100 magnétosomes, de préférence 10-60, et munis d'une charge superficielle cationique.

5. Magnétosomes selon la revendication 1 à 4, se caractérisant par le fait que des anticorps ou des médicaments sont liés en plus par covalence, le cas échéant par l'intermédiaire de groupes réactifs adéquats, à la membrane des magnétosomes.

6. Magnétosomes selon la revendication 1 à 5, se caractérisant par le fait qu'ils se présentent empaquetés dans des liposomes.

7. Magnétosomes selon la revendication 1 à 6, se caractérisant par le fait qu'ils se présentent empaquetés dans des liposomes classiques, des liposomes dits "Stealth R", des systèmes micellaires, des immunoliposomes, des liposomes cationiques ou des liposomes fusogènes.

8. Magnétosomes selon la revendication 1 à 4 et 6 à 7, se caractérisant par le fait qu'ils présentent en plus sur leur surface des anticorps spécifiques liés chimiquement.

9. Magnétosomes selon la revendication 1 à 4 et 6 à 7, se caractérisant par le fait qu'ils contiennent en plus un ou plusieurs médicaments sous forme capsulée (capsulage).

**10.** Magnétosomes selon la revendication 1 à 4 et 6 à 7, se caractérisant par le fait qu'ils contiennent en plus des radionucléides sous forme capsulée (capsulage).

**11.** Magnétosomes selon la revendication 1 à 4 et 6 à 7, se caractérisant par le fait qu'ils s'unissent avec du matériel génétique (par ex. des plasmides) des gènes thérapeutiques, des antisense oligonucléotides, des ribozymes ou des substances diagnostiques de gène pour former des complexes cationiques appropriés au transfert génétique.

**12.** Procédé de fabrication de magnétosomes spécifiques conformément à la revendication 1 à 4, se caractérisant par le fait qu'on les isole à partir de la bactérie magnétique *magnetospirillum gryphiswaldense* en employant un milieu de culture simple qui ne contient aucun agent complexant pour le fer, tout en maintenant la concentration en oxygène dans le milieu à moins de 2 %, en ajoutant plus tard de l'acétate de sodium ainsi que du $FeSO_4$, en récoltant les cellules magnétiques par centrifugation et enfin en produisant après lyse cellulaire les magnétosomes en séparant les débris de cellule et la sève de cellule avec un aimant permanent dans une colonne à fractionner magnétique.

**13.** Emploi de magnétosomes spécifiques conformément à la revendication 1 à 4 et 6 à 7 en tant que substance de contraste RMN.

**14.** Emploi de magnétosomes spécifiques conformément à la revendication 1 à 5 pour la fabrication de médicaments destinés au purging ("pêche à la cellule malade").

**15.** Emploi de magnétosomes spécifiques conformément à la revendication 1 à 4 et 6 à 7 pour la fabrication de substances permettant le diagnostic de tumeurs voire la lymphographie, de processus inflammatoires, de la sclérose en plaque, de la maladie d'Alzheimer et de la maladie de Parkinson.

**16.** Emploi de magnétosomes spécifiques conformément à la revendication 1 à 10 pour la fabrication de médicaments contre les tumeurs, les processus inflammatoires ainsi que les troubles du métabolisme.

pro          post 5min

post 24h     post 48h

Abb. 1